# EUROPEAN PATENT APPLICATION

(11) **EP 4 666 992 A1**
(43) Date of publication of application: **24.12.2025**
(21) Application number: 24775081.3
(22) Date of filing: 26.02.2024
(51) Int. Cl.: A61H 39/04, A61N 5/06

(54) **HAND ACUPOINT STIMULATION DEVICE USING HAND ACUPUNCTURE THERAPY**

(30) Priority: 21.03.2023 KR 20230036741
(71) Applicant: Samter Co., Ltd, Yongin-si, Gyeonggi-do 17036 (KR); Lee, Cheol Yun, Yongin-si, Gyeonggi-do 17036 (KR)
(72) Inventor: LEE, Cheol Yun, Yongin-si, Gyeonggi-do 17036 (KR)
(74) Representative: Isarpatent
(86) International application number: PCT/KR2024/002425
(87) International publication number: WO 2024/196033

(57) **Abstract**

The present invention is for providing a hand acupoint stimulation device using hand therapy that can simultaneously have a far-infrared effect, a warming effect, and an acupressure effect, the hand acupoint stimulation device including a housing having an open top surface and an accommodation space formed therein, an acupoint plate coupled to an opening in the top surface of the housing, and a far-infrared generator accommodated in the accommodation space of the housing, wherein a plurality of acupoint stimulators that correspond to different combinations of a plurality of acupoints according to different health conditions of a user are provided on the acupoint plate.

## Description

### [Technical Field]

The present invention relates to a hand acupoint stimulation device using hand therapy, and more particularly, to a hand acupoint stimulation device using hand therapy that can stimulate hand acupoints according to a health state of a user by using protrusions and the like that can have a far-infrared effect, a warming effect, and an acupressure effect.

### [Background Art]

Hand therapy, a unique Korean complementary and alternative therapy, views the hand as a microcosm of the human body and stimulates a body part that is in pain or has a disability by finding an acupoint on the hand that corresponds to the body part in order to reduce pain and improve function.

In addition, hand therapy is a method applying hand acupuncture, hand moxibustion, push rods, etc., to the hand and may be seen as a complementary and alternative therapy that is easy and convenient to apply, has no side effects, has rapid and long-lasting effects, and can be easily learned and used to self-manage pain, and it is known to be more effective when two therapies are applied in combination than when one therapy is applied alone.

Among different therapies, the hand acupuncture therapy involves inserting a needle at a depth of approximately 1 mm into an acupoint of the corresponding part, and although there is almost no possibility of side effects due to needle insertion, there is a risk of infection if a used needle is shared among multiple people without being properly sterilized, and in particular, there is a problem that needle insertion is difficult to perform on individuals with trypanophobia, a fear of sharp objects like needles.

Accordingly, development of electroacupuncture or the like that stimulates by passing a weak current is underway, and for example, Korean Patent Registration No. 10-1272904 (hereinafter referred to as "Patent Document 1") discloses a mouse that stimulates acupoints on the palm by having electrodes formed to allow microcurrent to flow through the hand of a user and applying microcurrent thereto.

**In** addition, the hand moxibustion therapy is a therapy that helps prevent, manage, and recover from a disease by burning wormwood placed on an acupoint and promoting metabolism and physiological functions through thermal stimulation, but there are disadvantages that the moxibustion therapy using wormwood carries the risk of burns and may cause discomfort due to smoke and moxibustion fumes, and in particular, it is not possible to maintain a constant temperature for a long time.

Accordingly, Korean Patent Registration No. 10-0162632 (hereinafter referred to as "Patent Document 2") discloses an electronic thermotherapeutic instrument that can produce a moxibustion effect due to heat generated after attaching a heating element to an acupoint on the human body and applying power to the heating element.

However, Patent Document 1 has a disadvantage that the scope of application is very limited because it is intended to alleviate pain in the wrists, fingers, or the like that results from a user using a computer mouse for a long time, and the electronic thermotherapeutic instrument of Patent Document 2 has disadvantages that it is inconvenient and cumbersome to use because for use of the electronic thermotherapeutic instrument, a double-sided tape should be used to attach the heating element to the acupoint on the human body, and the heat generated in Patent Document 2 is superficial heat energy and does not reach deep into the body.

Meanwhile, far-infrared rays are infrared rays with long wavelengths among infrared rays, and far infrared energy that has high penetrability into tissue of the human body and is transmitted deeper into the human body than heat energy transmitted only to the surface of the human body is known to be more efficient and less likely to cause burns than superficial heat energy of the same temperature.

**In** addition, far-infrared rays reach deep into the human body or material and are absorbed, resonate with the material's own wavelength, and activate the material's molecular motion, and in particular, since the human body is mostly composed of water and protein and molecular motion is activated by far-infrared rays, far-infrared rays are known to promote metabolism and blood circulation.

### [Disclosure]

### [Technical Problem]

The present invention has been devised to address the above-described problems of the related art and is directed to providing a hand acupoint stimulation device using hand therapy that can simultaneously have a far-infrared effect, a warming effect, and an acupressure effect.

The present invention is also directed to providing a hand acupoint stimulation device using hand therapy that is easy and convenient to use and can be reused with ease even when temporarily paused in the middle of use.

### [Technical Solution]

A hand acupoint stimulation device using hand therapy of the present invention includes a housing having an open top surface and an accommodation space formed therein, an acupoint plate coupled to an opening in the top surface of the housing, and a far-infrared generator accommodated in the accommodation space of the housing, wherein a plurality of acupoint stimulators that correspond to different combinations of a plurality of acupoints according to different health states of a user are provided on the acupoint plate.

In addition, in the hand acupoint stimulation device using hand therapy of the present invention, a hand placing part drawn in the shape of a hand may be provided on an upper surface of the acupoint plate, and the plurality of acupoint stimulators may be provided on the hand placing part.

In addition, in the hand acupoint stimulation device using hand therapy of the present invention, the plurality of acupoint stimulators may each include an acupressure part protruding from the upper surface of the acupoint plate and an acupoint stimulation hole part passing through the acupressure part and the acupoint plate.

In addition, in the hand acupoint stimulation device using hand therapy of the present invention, the acupoint stimulation hole part may have a hole diameter that gradually increases from the upper surface of the acupoint plate toward a lower surface thereof.

In addition, in the hand acupoint stimulation device using hand therapy of the present invention, an acupoint plate support part for supporting the acupoint plate may be provided on upper sides of sidewall parts of the housing, the acupoint plate may be coupled to the opening in the top surface of the housing while in contact with the acupoint plate support part, and the acupoint plate may be replaceable.

In addition, in the hand acupoint stimulation device using hand therapy of the present invention, at least one load sensor may be provided on an upper surface of the acupoint plate support part, a first temperature sensor and a second temperature sensor may be provided on inner surfaces of the sidewall parts of the housing, the first temperature sensor may be provided on an inner surface of a sidewall part of one side surface, and the second temperature sensor may be provided on an inner surface of a sidewall part of another side surface that faces the sidewall part of the one side surface.

In addition, the hand acupoint stimulation device using hand therapy of the present invention may further include a controller, and in a state in which the load sensor provided on the upper surface of the acupoint plate support part is in contact with the acupoint plate, the controller may control the far-infrared generator to operate when a load detected by the load sensor increases.

In addition, in the hand acupoint stimulation device using hand therapy of the present invention, the controller may stop an operation of the far-infrared generator when temperatures measured by the first temperature sensor and the second temperature sensor reach a predetermined highest temperature and may control the far-infrared generator to re-operate when the temperatures measured by the first temperature sensor and the second temperature sensor reach a predetermined lowest temperature.

In addition, in the hand acupoint stimulation device using hand therapy of the present invention, the controller may determine that a failure has occurred in the far-infrared generator when a difference between the temperatures measured by the first temperature sensor and the second temperature sensor deviates from a predetermined difference range.

### [Advantageous Effects]

According to the present invention, a far-infrared effect, a warming effect, and an acupressure effect can be simultaneously produced by far infrared rays and heat generated by a far-infrared generator and acupressure parts of acupoint stimulators.

In addition, according to the present invention, since a user only needs to operate a far-infrared generator by placing his or her hand on a hand shape drawn on a hand placing part on an acupoint plate, a hand acupoint stimulation device using hand therapy of the present invention is easy and convenient to use, and reusing the hand acupoint stimulation device is not cumbersome even when the hand acupoint stimulation device is temporarily paused in the middle of use.

In addition, according to the present invention, since a far-infrared generator starts to operate only when a user places his or her hand on a hand placing part on an acupoint plate, and heat generated by the far-infrared generator is adjusted within a predetermined temperature range, it is possible to prevent burns caused by low temperatures.

Further, according to the present invention, since a hand acupoint stimulation device using hand therapy of the present invention is configured to be portable, the hand acupoint stimulation device can be easily and conveniently carried and used in any place, and in particular, can be used while attached to a massage chair, sofa, or the like.

### [Description of Drawings]

FIG. 1 is a perspective view of a hand acupoint stimulation device according to the present invention.
FIG. 2 is a view for describing a structure of a housing in the hand acupoint stimulation device according to the present invention.
FIG. 3 is a cross-sectional view along line A-A of FIG. 1 and is a view showing an example of using a heat generating part including a ceramic layer as a far-infrared generator.
FIG. 4 is a cross-sectional view along line A-A of FIG. 1 and is a view showing an example of using a far-infrared generating light emitting diode (LED) as a far-infrared generator.
FIG. 5 is a schematic block diagram for describing an operational relationship of the hand acupoint stimulation device according to the present invention.
FIG. 6 is a view for describing various examples of an acupoint plate having acupoint stimulators formed thereon according to different health states of a user.
FIG. 7 is a view for describing a use example of the hand acupoint stimulation device according to the present invention.

### [Modes of the Invention]

Specific embodiments of a hand acupoint stimulation device using hand therapy according to the present invention (hereinafter referred to as "hand acupoint stimulation device") will be described in detail with reference to the accompanying drawings.

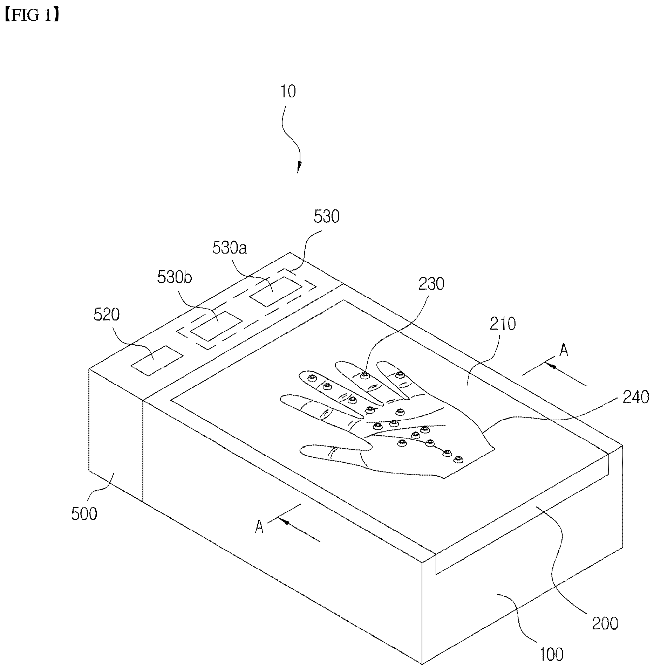

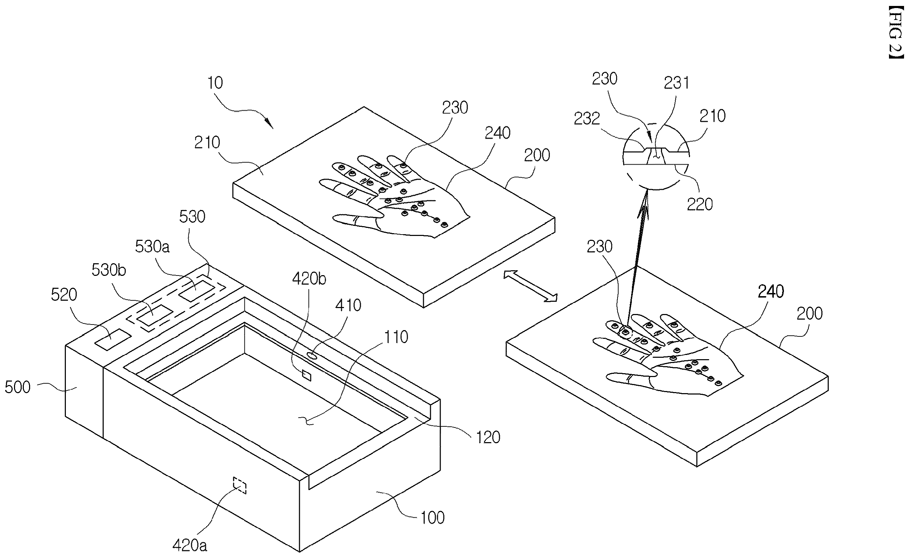

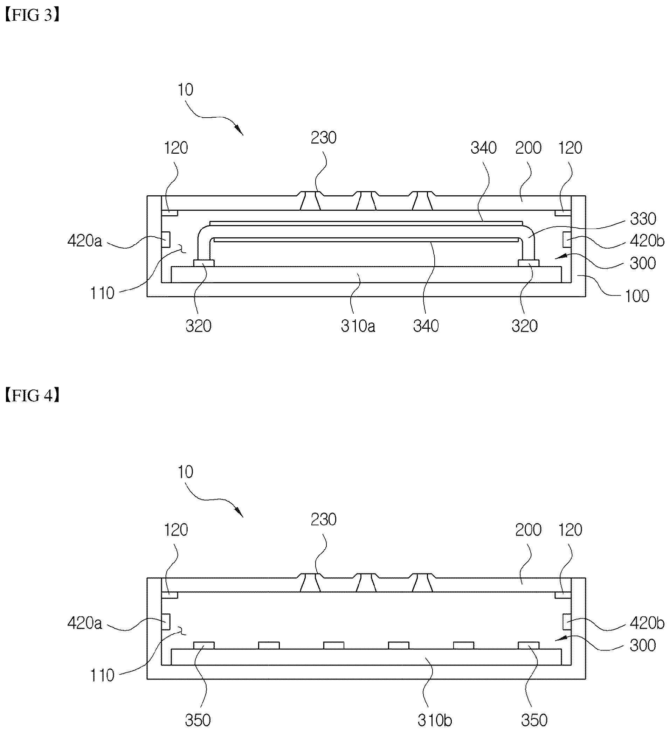

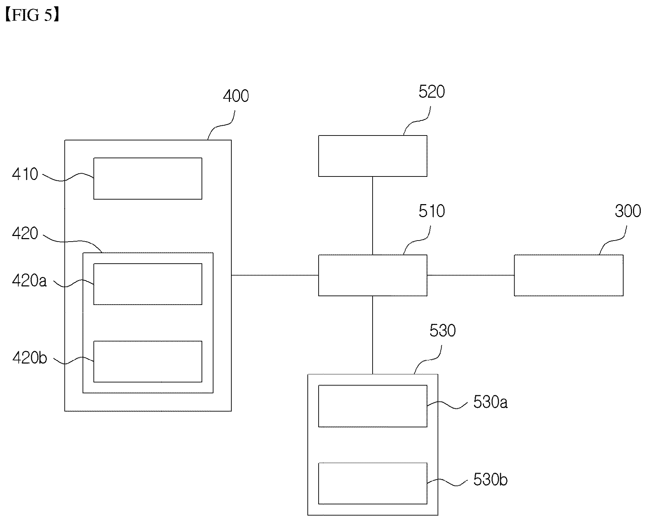

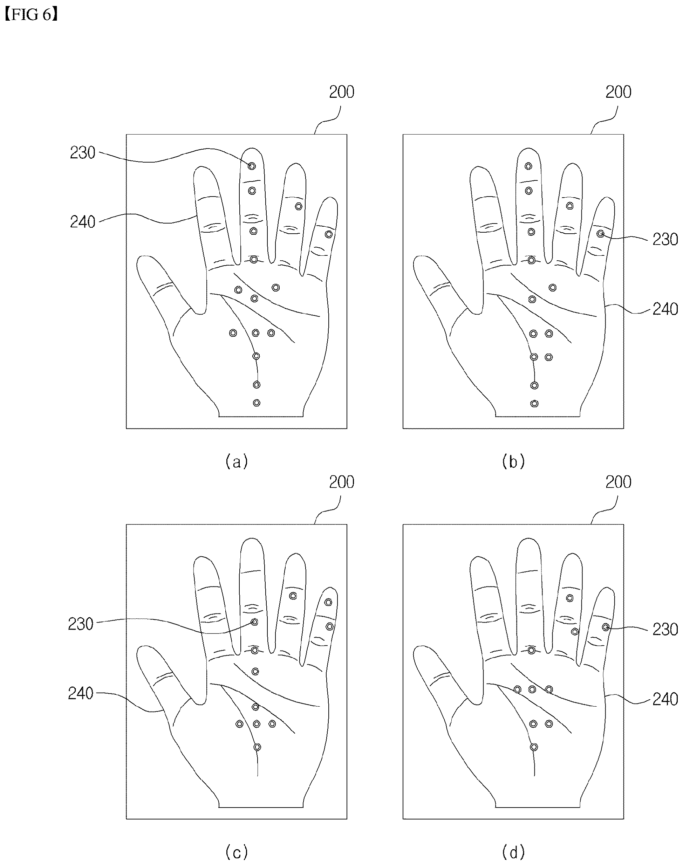

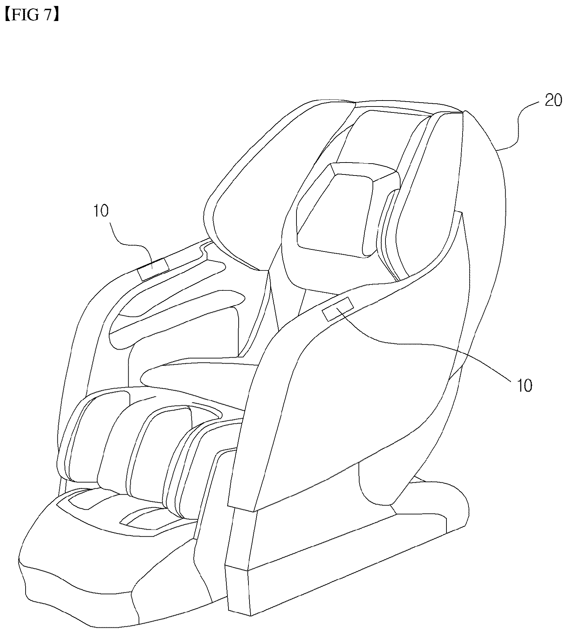

FIG. 1 is a perspective view of a hand acupoint stimulation device according to the present invention, FIG. 2 is a view for describing a structure of a housing in the hand acupoint stimulation device according to the present invention, FIG. 3 is a cross-sectional view along line A-A of FIG. 1 and is a view showing an example of using a heat generating part including a ceramic layer as a far-infrared generator, FIG. 4 is a cross-sectional view along line A-A of FIG. 1 and is a view showing an example of using a far-infrared generating light emitting diode (LED) as a far-infrared generator, FIG. 5 is a schematic block diagram for describing an operational relationship of the hand acupoint stimulation device according to the present invention, FIG. 6 is a view for describing various examples of an acupoint plate having acupoint stimulators formed thereon according to different health states of a user, and FIG. 7 is a view for describing a use example of the hand acupoint stimulation device according to the present invention.

First, as illustrated in FIGS. 1 to 5, a hand acupoint stimulation device 10 according to the present invention includes a housing 100 having an open top surface and an accommodation space 110 formed therein, an acupoint plate 200 coupled to an opening in the top surface of the housing 100, a far-infrared generator 300 accommodated in the accommodation space 110 of the housing 100, a sensor part 400 provided in the housing 100, and a control box 500 provided on one side of the housing 100.

The housing 100 is formed in the shape of a box that has the open top surface and the accommodation space 110 formed by a bottom part and sidewall parts of front, rear, left, and right surfaces, and although the housing 100 is illustrated as being formed in the shape of a quadrangular box in FIG. 1 to 4, the housing 100 is not limited thereto and may be formed in the shape of a circular box or an elliptical box, for example.

A sidewall part of one surface among the front, rear, left, and right surfaces of the housing 100 is cut by as much as a thickness of the acupoint plate 200, and a step is formed between the sidewall part of the one surface and a sidewall part of another surface that is not cut.

**In** addition, an acupoint plate support part 120 that supports a lower surface 220 of the acupoint plate 200 is provided on upper sides of inner side surfaces of the sidewall parts of the front, rear, left, and right surfaces.

In addition, the acupoint plate support part 120 that supports the lower surface 220 of the acupoint plate 200 may be provided only on an upper side of an inner side surface of the sidewall part of the other surface that is not cut, and at this time, the cut sidewall part may also serve to support the lower surface 220 of the acupoint plate 200.

The acupoint plate support part 120 may be formed to protrude inward from the inner side surfaces of the sidewall parts, and a height from an upper surface of the acupoint plate support part 120 to upper ends of the sidewall parts may match the thickness of the acupoint plate 200.

Next, the acupoint plate 200 has the shape of a plate that matches the top opening of the housing 100, the acupoint plate 200 is able to be coupled to and separated from the top opening of the housing 100 and is supported by the acupoint plate support part 120 and/or the cut sidewall part of the housing 100 when the acupoint plate 200 is coupled to the top opening of the housing 100, and airtightness in the housing 100 may be maintained by coupling between the acupoint plate 200 and the top opening of the housing 100.

**In** this way, since the acupoint plate 200 is supported by the acupoint plate support part 120 and/or the cut sidewall part of the housing 100, the acupoint plate 200 can be separated by being lifted or being slid toward the cut sidewall part and can be replaced with another acupoint plate 200 as shown in FIG. 2.

**In** addition, the acupoint plate 200 includes a hand placing part 240 drawn in the shape of a hand on an upper surface 210 of the acupoint plate 200 and a plurality of acupoint stimulators 230 formed at positions of predetermined acupoints according to health states of a user in the hand shape of the hand placing part 240.

That is, the acupoints are acupoints according to well-known Koryo hand therapy, and the plurality of acupoint stimulators 230 are provided to correspond to different combinations of a plurality of acupoints to stimulate the different combinations of a plurality of acupoints determined by Koryo hand therapy, according to different health states of a user.

For example, as illustrated in FIG. 6, (a) shows the acupoint plate 200 on which the plurality of acupoint stimulators 230 are formed to stimulate a combination of acupoints of male fundamental formula according to Koryo hand therapy, (b) shows the acupoint plate 200 on which the plurality of acupoint stimulators 230 are formed to stimulate a combination of acupoints of female fundamental formula according to Koryo hand therapy, (c) shows the acupoint plate 200 on which the plurality of acupoint stimulators 230 are formed to stimulate a combination of acupoints in the case of minor gastrointestinal disturbance according to Koryo hand therapy, and (d) shows the acupoint plate 200 on which the plurality of acupoint stimulators 230 are formed to stimulate a combination of acupoints in the case of acute indigestion or gastritis according to Koryo hand therapy.

Meanwhile, the acupoint plates 200 illustrated in FIG. 6 are only some examples, various other acupoint plates 200 on which the plurality of acupoint stimulators 230 are formed to correspond to various other combinations of acupoints for predetermined diseases according to Koryo hand therapy may be present, various hand placing parts 240 may be drawn according to the hand size, left hand/right hand, etc., and various acupoint plates 200 having the plurality of acupoint stimulators 230 formed thereon may be present according to various hand placing parts 240.

**In** addition, as illustrated in FIG. 2, the acupoint stimulators 230 each include an acupressure part 232 protruding from the upper surface 210 of the acupoint plate 200 and an acupoint stimulation hole part 231 passing through the acupressure part 232 and the upper surface 210 and the lower surface 220 of the acupoint plate 200.

**In** addition, the acupoint stimulation hole part 231 is a through-hole through which the far infrared rays and heat generated by the far-infrared generator 300 pass to stimulate acupoints of a user, the acupoint stimulation hole part 231 may be formed as a hole having a circular cross-section, and a diameter of the acupoint stimulation hole part 231 may gradually increase from the upper surface 210 toward the lower surface 220.

That is, in the acupoint stimulation hole part 231, a hole passing through the acupressure part 232 may extend with the same diameter, and then a hole passing from the upper surface 210 of the acupoint plate 200 to the lower surface 220 thereof may extend with a diameter that gradually increases.

Accordingly, when the acupoint plate 200 selected according to a health state of a user is coupled to the top opening of the housing 100, and then the user places his or her hand on the hand shape drawn on the hand placing part 240 of the acupoint plate 200, pressure is applied to acupoints on the hand of the user and acupressure is performed by the acupressure parts 232 protruding from the acupoint stimulators 230, and simultaneously, the far infrared rays and heat generated by the far-infrared generator 300 stimulate the acupoints on the hand of the user through the acupoint stimulation hole parts 231, thereby obtaining effects that can replace the conventional hand acupuncture and hand moxibustion.

**In** addition, since the diameter of the acupoint stimulation hole part 231 gradually increases from the upper surface 210 of the acupoint plate 200 toward the lower surface 220 thereof, the far infrared rays and heat generated by the far-infrared generator 300 may be efficiently gathered and moved to the acupoint stimulation hole part 231 close to upper surfaces of the acupressure parts 232.

Next, the far-infrared generator 300 is a device that is accommodated in the accommodation space 110 of the housing 100 and generates far infrared rays and heat, and the far-infrared generator 300 is connected to a power supply through a controller 510 which will be described below, and is not particularly limited as long as it is a device generating far infrared rays and heat.

That is, as illustrated in FIG. 3, the far-infrared generator 300 may include a substrate part 310a, a heat generating part 330 connected by a socket 320 provided on the substrate part 310a, and ceramic layers 340 provided on at least an upper surface and a lower surface of the heat generating part 330.

At this time, the heat generating part 330 and the ceramic layers 340 may be provided in a size that corresponds to the hand placing part 240 of the acupoint plate 200.

**In** addition, as illustrated in FIG. 4, the far-infrared generator 300 may include a substrate part 310a and a plurality of far-infrared generating light emitting diodes (LEDs) 350 provided on the substrate part 310a.

At this time, the plurality of far-infrared generating LEDs 350 may be provided to evenly correspond to the hand placing part 240 of the acupoint plate 200.

Next, the sensor part 400 includes at least one load sensor 410 provided on the upper surface of the acupoint plate support part 120 of the housing 100 and a plurality of temperature sensors 420 provided on the inner surfaces of the sidewall parts of the housing 100.

The load sensor 410 is provided on the upper surface of the acupoint plate support part 120 of the housing 100, and when the acupoint plate 200 is coupled to the top opening of the housing 100, the load sensor 410 also comes into contact with the lower surface 220 of the acupoint plate 200.

Accordingly, in a state in which the acupoint plate 200 is coupled to the top opening of the housing 100, when the user places or removes his or her hand on or from the hand placing part 240 of the acupoint plate 200, a change in the load can be detected by the load sensor 410.

**In** addition, the temperature sensors 420 are for measuring temperatures in the housing 100 that are caused by the heat generated by the far-infrared generator 300 and include at least two temperature sensors 420a and 420b.

At this time, when a first temperature sensor 420a is provided on an inner surface of a sidewall part of one side surface, a second temperature sensor 420b may be provided on an inner surface of a sidewall part of another side surface that faces the sidewall part of the one side surface.

That is, when the first temperature sensor 420a is provided on an inner surface of a sidewall part of the left side surface, the second temperature sensor 420b may be provided on an inner surface of a sidewall part of the right side surface, and when the first temperature sensor 420a is provided on an inner surface of a sidewall part of the front side surface, the second temperature sensor 420b may be provided on an inner surface of a sidewall part of the rear side surface.

At this time, the first temperature sensor 420a and the second temperature sensor 420b may be provided at the same height at positions facing each other.

Next, the control box 500 is provided on one side of the housing 100 and has the controller 510 provided therein, and an input part 520 and a display part 530 are provided to be exposed through an upper surface of the control box 500.

The on/off of power, use time settings, etc., may be input through the input part 520, and the display part 530 includes a first display part 530a displaying a temperature of the inside of the housing 100 and a second display part 530b displaying the remaining time of the use time set through the input part 520.

At this time, the first display part 530a may separately display the temperatures detected by the first temperature sensor 420a and the second temperature sensor 420b.

**In** addition, the controller 510 controls the far-infrared generator 300 and the display part 530 based on information input through the input part 520 and information detected by the sensor part 400.

Next, an operation process of the hand acupoint stimulation device 10 according to the present invention will be described.

First, the acupoint plate 200 is coupled to the top opening of the housing 100. At this time, the acupoint plate 200 may be selected according to a health state of the user, and for example, when the user does not have any illness at a specific site and wants to enhance health and prevent diseases, a male user may select the acupoint plate 200 illustrated in (a) of FIG. 6, and a female user may select the acupoint plate 200 illustrated in (b) of FIG. 6. In addition, a user with minor gastrointestinal disturbance or heartburn may select the acupoint plate 200 illustrated in (c) of FIG. 6, a user with acute indigestion, gastritis, food poisoning, abdominal pain, or diarrhea may select the acupoint plate 200 illustrated in (d) of FIG. 6, and the acupoint plate 200 may be replaced with another acupoint plate 200 for use according to circumstances.

Next, the user turns on the power through the input part 520 and sets the use time. At this time, even when the user turns on the power and sets the use time, the far-infrared generator 300 does not operate, and the set use time does not start to count down.

Next, when the user places his or her hand on the hand placing part 240 of the acupoint plate 200, a change (that is, an increase) in the load is detected by the load sensor 410, and at this time, the controller 510 operates the far-infrared generator 300.

**In** addition, from this time on, the controller 510 allows the temperatures measured by the first temperature sensor 420a and the second temperature sensor 420b to be displayed on the first display part 530a and allows the time set by the user to start counting down and the remaining time of the time set by the user to be displayed on the second display part 530b.

In addition, when the user removes the hand from the hand placing part 240 of the acupoint plate 200 in the middle of use, the load sensor 410 detects a change (that is, a decrease) in the load, and at this time, the controller 510 stops the operation of the far-infrared generator 300. At this time, the temperatures displayed on the first display part 530a may be displayed as they are, and the remaining time displayed on the second display part 530b may be displayed in a paused state.

In addition, the controller 510 may perform control by repeatedly stopping the operation of the far-infrared generator 300 when the temperatures measured by the first temperature sensor 420a and the second temperature sensor 420b reach a predetermined temperature and re-operating the far-infrared generator 300 when the temperatures measured by the first temperature sensor 420a and the second temperature sensor 420b drop to a temperature lower than or equal to a predetermined temperature, and in this way, the temperature of the inside of the housing 100 can be maintained at a temperature within a predetermined range.

That is, warming therapy is generally known to be effective when a temperature of tissue is maintained in a range of 40 to 45 °C for 30 minutes or more, but there is a risk of burns caused by low temperatures when contact occurs for a longer period of time at a temperature of 44 °C or higher.

Accordingly, when a higher temperature of the temperatures measured by the first temperature sensor 420a and the second temperature sensor 420b reaches 43 °C, the controller 510 may stop the operation of the far-infrared generator 300, and when the higher temperature of the temperatures measured by the first temperature sensor 420a and the second temperature sensor 420b drops to 40 °C, the controller 510 may re-operate the far-infrared generator 300, and in this way, the temperature of the inside of the housing 100 can be controlled to be maintained at a temperature within a range of 40 to 43 °C.

Accordingly, the highest temperature for stopping the operation of the far-infrared generator 300 (for example, 43 °C) and the lowest temperature for re-operating the far-infrared generator 300(for example, 40 °C) may be preset.

In addition, when a large difference is shown between the temperatures measured by the first temperature sensor 420a and the second temperature sensor 420b, a therapeutic effect may decrease due to heat of a different temperature being supplied to each acupoint on the hand of the user, and the large difference may also indicate that there is an abnormality in the hand acupoint stimulation device 10.

That is, in the case of the far-infrared generator 300 using the heat generating part 330 illustrated in FIG. 3, there may be an abnormality in a portion of the heat generating part 330, and in the case of the far-infrared generator 300 using the plurality of far-infrared generating LEDs 350 illustrated in FIG. 4, there may be an abnormality in some of the plurality of far-infrared generating LEDs 350.

Accordingly, when the difference between the temperatures measured by the first temperature sensor 420a and the second temperature sensor 420b is large (for example, the temperature difference is 2 °C or more), the controller 510 determines that there is an abnormality in the hand acupoint stimulation device 10 and controls the abnormality to be notified to the user through the first display part 530a.

Accordingly, a reference difference that is determined as indicating a failure (for example, 2 °C) may be preset in relation to the temperature difference between the first temperature sensor 420a and the second temperature sensor 420b.

In addition, a battery (not illustrated) may be embedded in the control box 500.

Accordingly, the hand acupoint stimulation device 10 according to the present invention can be easily and conveniently carried and used in any place, and in particular, as illustrated in FIG. 7, can be used while attached to a massage chair 20, sofa, or the like.

### [Industrial Applicability]

The present invention relates to a hand acupoint stimulation device using hand therapy that can simultaneously have a far-infrared effect, a warming effect, and an acupressure effect, and has industrial applicability.

## Claims

1. A hand acupoint stimulation device using hand therapy, the hand acupoint stimulation device comprising:
a housing having an open top surface and an accommodation space formed therein;
an acupoint plate coupled to an opening in the top surface of the housing; and
a far-infrared generator accommodated in the accommodation space of the housing,
wherein a plurality of acupoint stimulators that correspond to different combinations of a plurality of acupoints according to different health states of a user are provided on the acupoint plate.

2. The hand acupoint stimulation device of claim 1, wherein:
a hand placing part drawn in the shape of a hand is provided on an upper surface of the acupoint plate; and
the plurality of acupoint stimulators are provided on the hand placing part.

3. The hand acupoint stimulation device of claim 1 or 2, wherein the plurality of acupoint stimulators each include an acupressure part protruding from the upper surface of the acupoint plate and an acupoint stimulation hole part passing through the acupressure part and the acupoint plate.

4. The hand acupoint stimulation device of claim 3, wherein the acupoint stimulation hole part has a hole diameter that gradually increases from the upper surface of the acupoint plate toward a lower surface thereof.

5. The hand acupoint stimulation device of claim 1 or 2, wherein an acupoint plate support part for supporting the acupoint plate is provided on upper sides of sidewall parts of the housing, the acupoint plate is coupled to the opening in the top surface of the housing while in contact with the acupoint plate support part, and the acupoint plate is replaceable.

6. The hand acupoint stimulation device of claim 5, wherein at least one load sensor is provided on an upper surface of the acupoint plate support part, a first temperature sensor and a second temperature sensor are provided on inner surfaces of the sidewall parts of the housing, the first temperature sensor is provided on an inner surface of a sidewall part of one side surface, and the second temperature sensor is provided on an inner surface of a sidewall part of another side surface that faces the sidewall part of the one side surface.

7. The hand acupoint stimulation device of claim 6, further comprising a controller,
wherein, in a state in which the load sensor provided on the upper surface of the acupoint plate support part is in contact with the acupoint plate, the controller controls the far-infrared generator to operate when a load detected by the load sensor increases.

8. The hand acupoint stimulation device of claim 6, further comprising a controller,
wherein the controller stops an operation of the far-infrared generator when temperatures measured by the first temperature sensor and the second temperature sensor reach a predetermined highest temperature and controls the far-infrared generator to re-operate when the temperatures measured by the first temperature sensor and the second temperature sensor reach a predetermined lowest temperature.

9. The hand acupoint stimulation device of claim 6, further comprising a controller,
wherein the controller determines that a failure has occurred in the far-infrared generator when a difference between the temperatures measured by the first temperature sensor and the second temperature sensor deviates from a predetermined difference range.
